## Europäisches Patentamt

## European Patent Office

## Office européen des brevets

(11) Veröffentlichungsnummer: **0 116 352**
**B1**

(12) **EUROPÄISCHE PATENTSCHRIFT**

(45) Veröffentlichungstag der Patentschrift: **18.07.90**

(51) Int. Cl.⁵: **A 61 M 1/02**

(21) Anmeldenummer: **84101108.3**

(22) Anmeldetag: **03.02.84**

(60) Teilanmeldung **89114626.8** eingereicht am **03/02/84**.

(54) Autotransfusionsgerät.

(30) Priorität: **10.02.83 DE 3304486**

(43) Veröffentlichungstag der Anmeldung:
**22.08.84 Patentblatt 84/34**

(45) Bekanntmachung des Hinweises auf die Patenterteilung:
**18.07.90 Patentblatt 90/29**

(84) Benannte Vertragsstaaten:
**AT BE CH DE FR GB IT LI LU NL SE**

(56) Entgegenhaltungen:
**FR-A-2 346 238**
**GB-A-2 063 108**
**US-A-3 743 097**
**US-A-3 845 765**
**US-A-3 896 733**

(73) Patentinhaber: **Rühland, Dieter, Prof. Dr. med.**
**Jungeblodtplatz 1**
**D-4400 Münster (DE)**

(72) Erfinder: **Rühland, Dieter, Prof. Dr. med.**
**Jungeblodtplatz 1**
**D-4400 Münster (DE)**

(74) Vertreter: **Schumacher, Horst, Dr. Dipl.-Phys.**
**Frühlingstrasse 43 (Ecke Holunderweg)**
**D-4300 Essen 1 (DE)**

Courier Press, Leamington Spa, England.

EP 0 116 352 B1

**Beschreibung**

Die Erfindung betrifft ein Autotransfusionsgerät für Blut oder der gleichen Körperflüssigkeit sowie ein Verfahren zum Betreiben eines Autotransfusionsgerätes gemäß den Oberbegriffen der Anspruche 1 bzw. 11.

Bei Operationen, z.B. aus dem Bereich der Herzchirurgie, Gefäßchirurgie, Unfallchirurgie oder Orthopädie kommt es häufig zu großen schnellen Blutverlusten, die heute zumeist noch durch Fremdblut ausgeglichen werden. Fremdblut ist jedoch geeignet, Krankheiten zu übertragen (z.B. Hepatitis). Weiterhin fehlen dem oft mehrere Wochen alten Fremdblut die Gerinnungselemente (Gerinnungsfaktoren und Blutplättchen), die durch Lagerung zerstört werden. Es ist deshalb dringend erforderlich, Geräte zur Verfügung zu haben, die geeignet sind, daß sich in den Körperhöhlen während einer Operation ansiedelnde Blut zurückzugewinnen und dem Patienten wieder zuzuführen, um den Fremdbluteinsatz zur verringern und die Blutgerinnungselemente weitgehend zu erhalten.

Es stehen zur Rückgewinnung von Eigenblut (Autotransfusion) für große Herzoperationen komplizierte, teure Herz-Lungen-Maschinen heute zur Verfügung, die das Blut des Patienten über einen Pumpmechanismus absaugen und über lange Schläuche wieder zuführen. Andere Geräte sammeln die Blutflüssigkeit, isolieren und waschen die roten Blutzellen und führen diese gewaschenen Blutzellen dem Körper wieder zu. Bei beiden Geräten werden die empfindlichen Blutzellen durch lange Saugwege geschädigt (Oberflächenkontakt) und bei dem weiten Gerät werden durch den Waschvorgang alle Gerinnungselemente und das Blutplasma entfernt. Diese Geräte sind zudem sehr teuer und daher nur in wenigen Zentren verfügbar.

Es sind daher weitere Geräte für die intraoperative Autotransfusion entwickelt worden. So ist aus der US—PS 4 047 526 ein Autotransfusionsgerät der eingangs genannten Art bekannt, bei dem das Blut zunächst in einem harten Behälter durch Unterdruck gesammelt und anschließend in einen mit dem harten Sammelbehälter bodenseitig lösbar verbundenen Faltbalg, mittels dessen ein Vakuum erzeugbar ist, durch welchen das Vakuum in dem darüberliegenden harten Behälter überwunden werden kann, überführt. Der mit Blut gefüllte Faltbalg wird dann von dem harten (formsteifen) Behälter gelöst und anschließend zur aktiven Rücktransfusion des Blutes verwendet.

In der US—PS 4 033 345 wird eine andere Ausführungsform eines Autotransfusionsgerätes mit einem formsteifen Zweikammersystem beschrieben, das einen innenliegenden verformbaren Beutel enthält, der mit der oberen Kammer, die zunächst das Blut sammelt, durch ein Rückschlagventil verbunden ist. Das Blut kann durch eine weitere Öffnung mit Rückschlagventil und Filtereinheiten durch Eindringen eines Druckmediums in den Raum zwischen der harten, zweiten Kammer und der äußeren Beuteloberfläche aktiv in den Patienten zurücktransfundiert werden. Die Überleitung des Blutes aus dem formsteifen Sammelbehälter in den flexiblen Beutel erfolgt durch wechselseitiges Anlegen von Über- und Unterdruck.

Bei einem aus der nachveröffentlichten DE—OS 32 18 561 bekannten, weiteren Autotransfusionsgerät handelt es sich um eine der zweiten Kammer der vorerwähnten US—PS 4 033 345 entsprechende Vorrichtung, mit der das Blut jedoch ohne Rückschlagventile durch eine Öffnung in der Kammerunterseite ausgesaugt werden muß und bei der weder ein Sieb noch ein ähnliches Blutfilter einsetzbar ist.

Schließlich ist aus der US—PS 4 014 329 ein weiteres Zweikammerautotransfusionsgerät bekannt, bei dem die erste Kammer nach dem gleichen Prinzip arbeitet, wie es in der US—PS 4 033 345 für die zweite Kammer beschrieben wurde, wobei hier aber das Blut durch seine Schwerkraft an der Kammerunterseite in die zweite Kammer mit Filter abfließt.

Mit diesen bekannten Autotransfusionsgeräten ist es zwar möglich, intraoperativ anfallendes Blut zu sammeln und in den Patienten zu retransfundieren, doch weisen sie eine Reihe entscheidender Nachteile auf: Bei den Mehrkammersystemen kommt das Blut mit einer großen Geräteoberfläche in Kontakt, was eine nachteilige Gerinnungsaktivierung und Bluttraumatisierung bewirkt. Ebenso erfolgt eine für die empfindlichen Blutzellen nachteilige, weitere Traumatisierung bei der Überführung von der einen in die andere Kammer, insbesondere wenn zwischen den Kammern Rückschlagventile eingesetzt sind. Soweit das Blut von unten in die Unterdruckkammer eingesaugt wird, wird das im Gerät bereits angesammelte Blut durch nachfolgend angesaugtes Blut in turbulente Bewegung versetzt, wobei mitangesaugte Luft und grobe Bestandteile, wie Blut, Koagel, Fettzellen und Knochensplitter, eine erhebliche Schaumbildung sowie Traumatisierung der Blutzellen bewirken. Beim Fehlen von Grobfiltern zum Zurückhalten von im Blut mitgeführten Stoffen besteht schließlich bei den herkömmlich an den Transfusionsbestecken vorgesehenen Feinfiltern eine latente Verstopfungsgefahr. Weiterhin erfordern die deckel- sowie bodenseitig mit Ein- bzw. Auslauföffnungen versehenen Blutsammelkammern eine im Hinblick auf das erforderliche Blutansaugvolumen beachtliche Bauhöhe, welche regelmäßig höher als das sehr kleine sterile Operations-Gebiet am Patienten ist; daher ergeben sich bei den bekannten Autotransfusionsgeräten regelmäßig Sterilitätsprobleme im sterilen Operations-Gebiet, die nur dadurch ausgleichbar sind, daß das Autotransfusionsgerät außerhalb des Sterilbereiches aufgestellt und ein längerer Saugweg in Kauf genommen wird. Hierdurch ist ein höherer Saugdruck erforderlich- und ein vermehrter Fremdkörperkontakt gegeben — beides bedeutet ein zusätzliches Trauma für das Blut. Bei Zweikammersystemen erfordert die Überleitung des Blutes nach der Aspiration in die zweite Kammer Zeit, die weder dem Anästhesisten, noch dem Chirurgen bei einem Massenanfall von Blut zur Verfügung steht; der Zeitfaktor ist besonders aber auch für den Patienten nachteilig, da er das Blut in diesem Falle besonders schnell wieder benötigt.

Aus der FR—R—23 46 238 ist eine Blutpumpe bekannt, deren Aufgabe darin besteht, eine Blut-/ Luftoberfläche bei der Blutaufnahme zu vermeiden. Zu diesem Zweck wird der Über- und Unterdruck zur Pumpenbetätigung ausschließlich auf eine der Blutsammelkammer bezüglich einer Membran gegenüberliegende Luftkammer ausgeübt, wobei sich die Einlauf- und Auslauföffnung für das Blut im Bodenteil des Pumpenbehälters befinden, während sich die Luftkammer ständig im Deckelteil befindet. Selbst wenn zusammen mit dem Blut versehentlich einmal etwas Luft angesaugt wird, wird diese im Normalfall nicht weiter transportiert und bildet auch keine, das nachströmende Blut schädigende, Blut-/Luftoberfläche, weil sich die Luft zwingendermaßen im höchsten Bereich des Bodenteiles sammelt und das weiter ein- und ausströmende Blut unterhalb dieser Oberfläche angesaugt und ausgestoßen wird, mit der Luft also nicht in Kontakt kommt. Zwischen der Zulaufleitung und der Ablaufleitung der unteren Kammer ist also eine permanente Flüssigkeitsverbindung gewährleistet. Bei einer derartigen Blutpumpe ist darauf zu achten, daß Strömungswiderstände, wie sie zum Beispiel ein Sieb in der Auslaufleitung sowie davor etwa sich ansammelnde Partikel darstellen würden, vermieden werden. Als Autotransfusionsgerät ist eine derartige Blutpumpe keinesfalls geeignet, weil mit dem Blut angesaugte Luft sowie Fette oder andere Partikel beim Reinfundieren in den Patienten nicht automatisch zurückgehalten werden können.

Davon ausgehend liegt der Erfindung die Aufgabe zugrunde, ein Autotransfusionsgerät der eingangs genannten Art zu schaffen, mit dem es möglich ist, das Blut auf kurzem Wege ohne Schädigung zu sammeln und schnell bei sicherer Funktionstüchtigkeit und ohne Schädigungsmöglichkeit für den Patienten in diesen zurückzutransfundieren; insbesondere sollen die vom Blut berührten Kontaktflächen möglichst klein sein und Engstellen sowie Schaumbildung vermieden werden und eine geringe Bauhöhe erreicht werden, die eine Anwendung im Sterilbereich des Patienten gestattet.

Als technische Lösung wird dafür ein Autotransfusionsgerät mit den Merkmalen des Anspruchs 1 und ein Verfahren mit den Merkmalen des Anspruchs 11 vorgeschlagen.

Die Erfindung beruht demnach auf dem Grundgedanken, im Deckelteil einer einzigen Blutsammelkammer sowohl die Bluteinlaßöffnung, als auch die Blutauslaßöffnung mit vorgeschaltetem Grobfilter anzuordnen, so daß das angesaugte Blut von oben in diese Kammer einströmt und ohne Schaumbildung an der Kammerwandung entlang nach unten fließt; bei der Retransfusion wird das Gerät auf den Kopf gestellt und das Blut fließt gefiltert durch Schwerkraft und/oder erhöhten Gasdruck in dem durch eine Membran von der Blutsammelkammer abgetrennten Bodenraum zurück zum Patienten; die vorangehende Blutansaugung wird dabei durch einen Unterdruck bewirkt, der entweder — bei gasdicht verschlossenem Bodenraum — an der Auslauföffnung, oder — bei gasdicht verschlossener Auslauföffnung — im Bodenraum angelegt wird; die Membran ist demnach nur an ihrem einzigen Rand zwischen Boden und Deckelteil festgelegt, wobei das Größenverhältnis zwischen Boden- und Deckelteil unerheblich ist und lediglich gewährleistet sein muß, daß sich die Membran alternierend sowohl an der Bodenteil- als auch an der Deckelteilwandung möglichst weitgehend anlegen kann, so daß abwechselnd der Boden und der Deckelraum ein minimales Restvolumen besitzen.

Die Einlauf- und Auslauföffnungen müssen bei Unterdruckanwendung im Deckelraum hinreichend weit beabstandet sein, um einen Blutübertritt in die angelegten Unterdruckquelle zu vermeiden.

Des erfindungsgemäße Autotransfusionsgerät weist eine Reihe wichtiger Vorteile auf: Das Blut wird in einer einzigen Kammer ohne verengende Querschnitte, ohne Schaumbildung unter kontrolliert erzeugbarem Druck gesammelt und vorgefiltert aus derselben Kammer unter Anwendung eines frei vorgebbaren Druckes mit der gewünschten Schnelligkeit direkt in den Patienten infundiert; hierfür ist nur eine sehr geringe Bauhöhe des unterdruckfesten Behälters erforderlich, so daß dieser jederzeit im Sterilbereich der Operation handhabbar ist. Zudem werden Dichtigkeitsprobleme im Bereich der drei Behälteröffnungen und im Zusammenhang mit der Membran dadurch vermieden, daß diese Öffnungen allesamt in der im wesentlichen starren Wandung des Deckel- bzw. Bodenteiles, und daher unabhängig vom Dichtungsrand der Membran, angeordnet sind; es braucht also lediglich der Membranrand in der funktionell dafür am besten geeigneten Weise zwischen Deckel- und Bodenteil festgelegt werden, während Durchlaßöffnungen mit Schlauchanschlüssen innerhalb der Membranfläche entfallen; hierdurch können die Membraneigenschaften und die Membranabdichtung besonders funktionsgerecht und unabhängig von der Ein- und Auslaßöffnung frei ausgewählt werden.

Es versteht sich, daß das erfindungsgemäße Autotransfusionsgerät auch derart benutzt werden kann, daß einem Patienten Blut entnommen und dieses unmittelbar anschließend einem anderen Patienten zugeführt wird.

Die Membran kann zwischen dem Boden- und Deckelteil der Flasche verschweißt oder mit einem verstärkten Rand als Dichtung zwischen Boden- und Deckelteil in einer Ringnut mittels Schraubverschluß lösbar eingeklemmt werden. Gemäß einer anderen Weiterbildung der Erfindung kann das Sieb, anstelle im Deckel eingeschweißt oder eingeklebt zu sein, auch in einem Deckfalz lösbar gehalten werden. Das Einklemmen des Membranrandes hat den Vorteil, daß die Dichtung zwischen der Membran und dem Behälter von dem in dem Boden- bzw. Deckelraum herrschenden Druck nicht belastet wird, so daß keine Gefahr besteht, daß der ausgeübte Über- oder Unterdruck die Dichtigkeit zwischen dem Membranrand und dem Behälter vermindert.

Nach einer weiteren Ausführungsform der Erfindung ist es möglich, als Membran einen aufblasbaren, mit seinem Mündungsrand gegenüber dem Rand der Öffnung für vom Behälterinneren verschiedenen Gasdruck im Bodenteil gasdicht festgelegten Ballon zu verwenden. Dieser ist zweckmäßig am Behälterboden fixiert.

Mit dem angesaugten Blut gelangen häufig auch Schaum- und Fetteile in den Deckelraum, weshalb das Sieb bevorzugt mit Abstand vor der Auslauföffnung im Behälter gehalten ist. Dadurch wird einerseits erreicht, daß die Membran bei Druckluftanwendung nur bis zur Anlage an das Sieb gedrückt werden und in der Auslaßöffnung daher nicht verletzt werden kann. Andererseits wird nach dem Umdrehen des Behälters, wenn also die Auslauföffnung nach unten weist, zwischen Sieb und Auslaufstutzen eine Restmenge Blut zurückgehalten, auf der die Fetteile und der Schaum schwimmen, die dann leicht verworfen werden können.

Durch einen entsprechend geformten Saugstutzen als Einlauföffnung wird die Körperflüssigkeit an der Innenwand des Deckelteils und der Membran so eingeleitet, daß sie atraumatisch herunterfließt, was gemäß einer Weiterbildung der Erfindung durch eine tangential zur Innenwand des Deckelteils mündende Einlauföffnung erreicht werden kann. Dieses glatt Einlaufen des Blutes wird unterstützt, wenn die Einlauföffnung aus einem trompetenartig erweiterten oder T-förmigen Mundstück besteht, so daß die Strömung des in das Behälterinnere gelangenden Blutes verlangsamt ist.

Das erfindungsgemäße Autotransfusionsgerät schont das zu transfundierende Blut sowie den Patienten auf bestmögliche Weise: Während des Blutansaugens entweicht nämlich die mitangesaugte Luft durch den an der Auslauföffnung angeordneten Unterdruckanschluß und wird nicht traumatisierend durch das gesammelte Blut hindurchgesaugt; diese Verwendungsart hat gegenüber einem Unterdruckanschluß im Bodenraum — bei dem die Bluttraumatisierung ebenfalls vermieden wird — den Vorteil, daß größere Mengen mitangesaugter Luft die Blutaufnahmekapazität des Autotransfusionsgerätes nicht vermindern. Nach beendeter Aspiration eventuell noch vorhandene Luft im Deckelraum kann vor der Retransfusion durch Einbringung von Druckfluid in den Bodenraum zunächst ausgetrieben werden, wobei Blutgerinsel und grobe Gewebeteile von dem Sieb zurückgehalten werden.

Der Behälter und die Membran können unabhängig voneinander aus verschieden elastischen, blutfreundlichen Werkstoffen, wie Polyurethan, Polyvinylchlorid, Silicon-Gummi, Polyäthylen u.ä., hergestellt werden.

Natürlich ist es möglich, gerinnungshemmende Flüssigkeiten in den Behälter einzubringen, ohne diese durch den Blutsauger aufsaugen zu müssen. Hierzu bietet sich ein der Einlaßöffnung vorgeschalteter Zwei- oder Dreiwegehahn oder eine weitere Einlaßöffnung im Deckelteil an. Der Dreiwegehahn, bzw. ein T- bzw. Y-Stück vor der patientenseitigen Einlauföffnung ermöglicht zudem ein gleichzeitiges Anschließen von zwei Autotransfusionsgeräten an die Blutansaugleitung, so daß entweder mit erhöhter Saugleistung bzw. ununterbrochen Blut angesaugt werden kann, indem mindestens ein Autotransfusionsgerät ständig an die Ansaugleitung angeschlossen und das andere Gerät nach Kapazitätsausschöpfung abgeklemmt und durch ein frisches Gerät ausgetauscht werden kann.

Mit dem erfindungsgemäßen Autotransfusionsgerät ist es also möglich, mehrere wichtige Kriterien gemeinsam zu erfüllen, nämlich schonende Aspiration, aktive Retransfusion, Zurückhaltung aller groben Schadstoffe, Zurückhaltung von Fett und Schaum sowie mögliche Wiederverwendbarkeit, verbunden mit einer sicheren und ständigen Funktionsbereitschaft.

Es versteht sich, daß die Merkmale der Unteransprüche in beliebig sinnvoller Kombination mit den Merkmalen der übergeordneten Ansprüche ebenfalls den Gegenstand der Erfindung bilden.

Weitere Einzelheiten, Merkmale und Vorteile des Gegenstandes der Erfindung ergeben sich aus der nachfolgenden Beschreibung der zugehörigen Zeichnung, in der eine bevorzugte Ausführungsform eines erfindungsgemäßen Autotransfusionsgerätes dargestellt worden ist. In der Zeichnung zeigen:

Fig. 1 ein Autotransfusionsgerät im Längsschnitt;

Fig. 2 ein anderes Autotransfusionsgerät in perspektivischer Ansicht;

Fig. 3 dasselbe Autotransfusionsgerät im Längsschnitt entlang der Linie III—III gemäß Fig. 2 in vergrößerter Darstellung;

Fig. 4 dasselbe Autotransfusionsgerät in der Innenansicht A gemäß Fig. 2 sowie

Fig. 5 ein anderes Autotransfusionsgerät im Längsschnitt in Teilansicht.

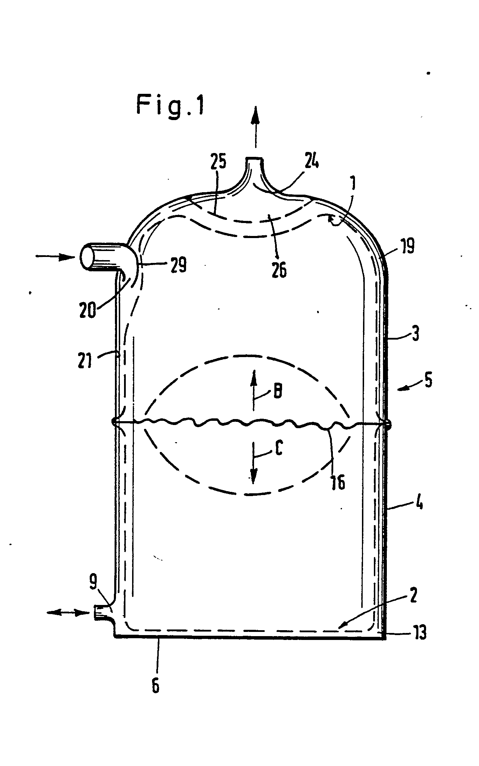

Fig. 1 zeigt den wesentlichen Teil eines Autotransfusionsgerätes in Form eines im ganzen mit 5 bezeichneten, aus sterilisierbarem Kunststoff oder Glas bestehenden Behälter, der als Einmal-Behälter ausgeführt sein kann. Der Behälter weist einen zylindrischen oder rechtwinkligen Querschnitt auf und besteht aus einem Bodenteil 4 und einem mit dessen oberem Rand fluiddicht verbundenen Deckelteil 3. Der Behälter 5 ist unterdruckstabil und über eine Auslauföffnung 24 im Deckelteil 3 oder eine Öffnung 9 im Bodenteil 4 evakuierbar. Im Deckelteil 3 ist in ausreichendem Abstand von der Auslauföffnung 24 eine Einlauföffnung 24 im Bereich eines trompetenförmigen Mundstücks 29 tangential zur Innenwand 21 des Deckelteils 3 angeordnet, so daß vom Patienten angesaugte Körperflüssigkeit an dieser Innenwand durch Gravitation ohne Schaumbildung hinabläuft. Ein Sieb 25 zum Zurückhalten von im Blut mitgeführten Grobstoffen deckt die Auslauföffnung 24 mit Abstand vom Behälterinnenraum her ab. Die Öffnung 9 ist gasdicht verschließbar und ermöglicht nach Entfernen des Verschlusses das Eindringen atmosphärischer Luft oder von Druckluft in das Behälterinnere. Eine durch Druck verformbare, für die Körperflüssigkeit undurchlässige Membran 16 ist zwischen dem Bodenteil 4 und dem Deckelteil 3 randseitig gasdicht derart festgelegt, daß sie den Behälter 5 in einen gasgefüllten Bodenraum 13 und einen demgegenüber abgedichteten Deckelraum 19 — zum Aufnehmen der Körperflüssigkeit — unterteilt. Unter dem Einfluß eines Fluiddruckes kann sich die Membran 16, wie gestrichelt dargestellt, an die Innenkontur 1 des Deckelteils 3 sowie alternierend an die Innenkontur 2 des Bodenteils 4 im wesentlichen anlegen. Selbstverständlich sind alle Zwischenpositionen der Membran 16 zwischen diesen beiden Extrempositionen einnehmbar, was durch weitere gestrichelte Linien und die Richtungspfeile B und C dargestellt ist.

Die Verbindung zwischen dem Membranrand und dem Boden- und Deckelteil kann durch Kunststoffverschweißen unter Bildung eines einteiligen Einmal-Behälters erfolgen.

Bei völlig mit Blut gefüllten Behältern nimmt die Membran 16 im wesentlichen die Form der Innenkontur 2 ein. Dann wird die Zulaufleitung vom Patienten dicht verschlossen und ggf. patientenseitig abgeklemmt. Nach dem Entfernen einer nicht dargestellten Vakuumleitung, die entweder an die Auslaßöffnung 24 oder an die Öffnung 9 angeschlossen ist, kann durch Einlassen von Druckluft durch die Öffnung 9 und Anschließen eines Retransfusionsbesteckes an die Öffnung 24 die im Deckelraum 3 und den nachgeordneten Systembereichen noch vorhandene Luft ausgetrieben werden. Dabei hält das Sieb 25 Grobbestandteile zurück. Nach dem Auf-den-Kopf-Drehen des Behälters 5 haben sich nach einer Beruhigungszeit alle Leichtstoffe des Blutes, wie noch nicht ausgetriebene Luftbläschen und Fettzellen, unter der — dann oben liegenden — Membran 16 gesammelt. Beim anschließenden Einlassen von Umgebungs- oder Druckluft durch die Öffnung 9 strömt das Blut so lange zum Patienten zurück, bis die Membran 16 sich an die Kontur 1 des Deckelteils 3 sowie an das Sieb 24 ganz angelegt hat. Dabei verbleiben zwischen Membran 16 und Sieb 25 im Deckelraum 19 nur noch vom Sieb zurückgehaltene Grobbestandteile, während Feinbestandteile, wie die erwähnten Luftblasen oder Fettzellen dann konzentriert im Raum 26 zwischen der Auslauföffnung 24 und dem Sieb 25 angesammelt sind und nicht mehr zum Patienten gelangen können.

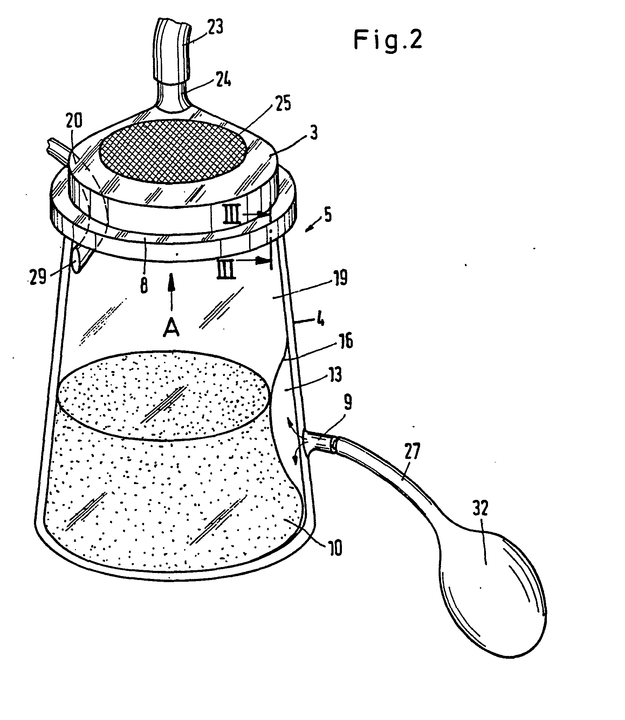

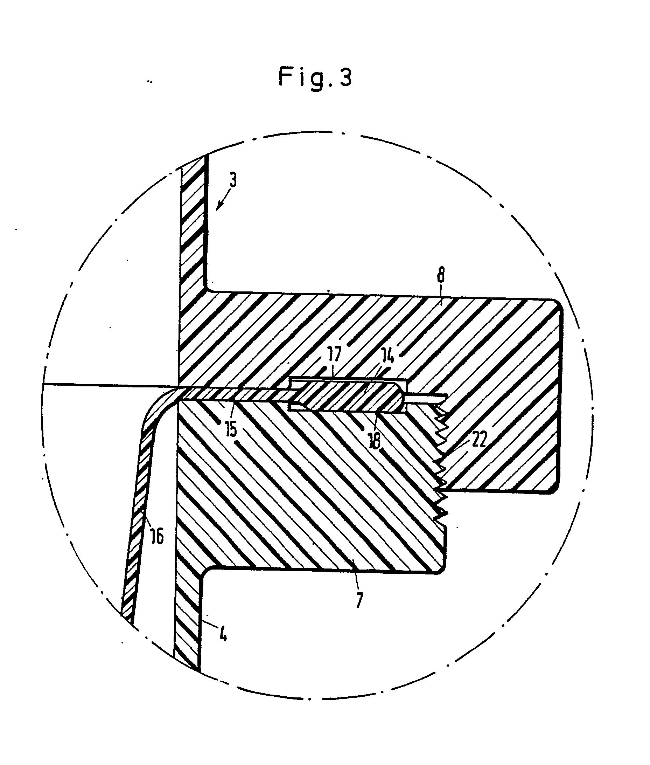

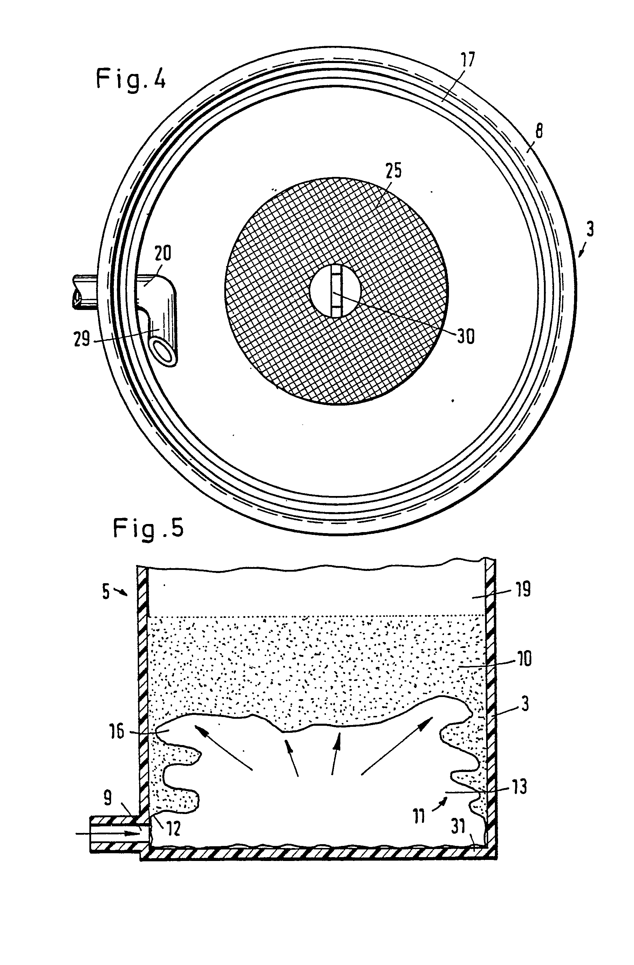

Gemäß Fig. 2 kann ein wiederverwendbares Autotransfusionsgerät einen verbreiterten Fuß 6 (auch bei Fig. 1 möglich) sowie einen radial abstehenden Schraubrand 7 am offenen Ende des Bodenteils 4 und einen damit korrespondierenden umlaufenden Rand 8 am offenen Ende des Deckelteils 3 aufweisen. In der Mitte des Deckelteils 3 ist an die Auslauföffnung 24 ein Auslaufstutzen angeformt, auf den ein Blutabflußschlauch 23 aufgesteckt ist. In der Nähe des Randes 8 ist durch das Deckelteil 3 ein Einlaufstutzen mit einer Einlauföffnung 20 durchgeführt, auf den noch eingegangen wird. Die Innenfläche des Deckelteils 3 weist einen ringförmigen Absatz auf, in welchen ein scheibenförmiges Sieb 25 eingesetzt ist. Der Absatz ist so gestaltet, daß das Sieb im Deckelteil 3 im Klemmsitz gehalten ist und bei Bedarf ausgewechselt werden kann. Zur besseren Handhabung ist am Sieb 25 ein Handgriff 30 (Fig. 4) angeformt. Zwischen Rand 8 und Schraubrand 7 ist der Rand (Randwulst) 14 einer dünnen Membran 16 so eingeklemmt (siehe im einzelnen Fig. 3), daß die Membran beutelförmig im Inneren des Bodenteils 4 frei hängt. Die Membran 16 besteht aus weichem, z.B. gummiartigem, Sterilisierbaren Material, dessen Form einer Druckbelastung ohne weiteres nachgibt. Auf die Anschlußtülle bei Öffnung 9 des Bodenteils 4 kann eine Verschlußkappe aufgesetzt sein; sie kann entfernt werden und durch einen von einer Druckquelle 32 in Form eines handbetätigten Balles kommenden Druckschlauch 27 ersetzt werden.

Gemäß Ausschnitts-Darstellung der Fig. 3 weist das Deckelteil 3 einen umfangsmäßig vorstehenden Rand 8 auf, der am äußeren, abgewinkelten Ende mit einem Gewinde 22 versehen ist. Der umfangsmäßig vorstehende Schraubrand 7 des Bodenteils 4 ist an seiner Außenfläche mit einem korrespondierenden Schraubgewinde versehen, so daß das Deckelteil auf das Bodenteil aufgeschraubt und von diesem zum Ersatz der Membran 16 gelöst werden kann. Der Rand 8 besitzt an seiner nach unten weisenden Fläche eine Ringnut 17, und im dargestellten Ausführungsbeispiel ist die nach oben weisende Fläche des Schraubrandes 7 mit einer gegenüberliegenden Ringnut 18 versehen. Der Rand 14 der Membran 16 ist derart verstärkt, daß er in den durch die Ringnuten definierten Raum eingefangen ist, wenn Deckelteil 3 auf Bodenteil 4 aufgeschraubt ist.

Wie Fig. 3 besonders deutlich zeigt, wird der an den verdickten Rand 14 anschließende Randabschnitt 15 der Membran 16 beim Aufschrauben des Deckelteils auf das Bodenteil zwischen Rand 8 und Schraubrand 7 eingeklemmt. Diese Art der Verankerung des Membranrandes an dem Behälter 5 hat den besonderen Vorteil, daß der zum Herausdrücken des Blutes 10 (Fig. 2) aus dem Deckelraum 19 angewandte positive Druck im Bodenraum 13 keinen Einfluß auf die Festigkeit der Klemmung des Randabschnitts 15 der Membran 16 hat. Damit ist sichergestellt, daß der Bodenraum 13 vom Deckelraum 19 stets gasdicht abgeschlossen bleibt. Die Funktion des Autotransfusionsgerätes gemäß Fig. 2 entspricht im übrigen der im Zusammenhang mit Fig. 1 beschriebenen Funktion, wobei nunmehr nach erfolgter Retransfusion die vier Hauptbaugruppen (Bodenteil, Deckelteil, Sieb und Membran) getrennt, gereinigt und sterilisiert bzw. ersetzt werden können, also daß Mehrfachverwendung möglich ist.

Es versteht sich, daß das Bodenteil 4 im Extremfall die Form eines flaschenähnlichen Behälters mit relativ kleiner Deckelöffnung aufweisen kann, wobei ein relativ kleines Deckelteil 3 randseitig eine beutelförmige Membran 16 am Öffnungsrand des Bodenteils 4 festlegt und eine Einlaßöffnung sowie eine Auslaßöffnung mit Sieb aufweisen muß.

Fig. 5 zeigt eine alternative Ausführung der Erfindung, bei der die Membran 16 die Form eines aufblasbaren Ballons 11 hat. Der Ballon 11 ist am Boden 31 des Behälters 5 in geeigneter Weise, beispielsweise durch Verkleben, fixiert. Der Mündungsrand 12 des aufblasbaren Ballons 11 ist in der bodennahen Öffnung 9 umlaufend und gasdicht befestigt. Hierzu kann der Mündungsrand 12 um eine Anschlußtülle der Öffnung 9 umgelegt und mit einer Steckkappe oder dgl. gehalten werden. Damit reduziert sich das Bodenteil auf diese Steckkappe — ebenso wie sich das Deckelteil (wie vorerwähnt) ebenfalls auf einen sehr kleinen Bereich des Behälters 5 reduzieren kann.

Die Gefahr einer Traumatisierung des Blutes 10 beim Einleiten in den Behälter 5 wird (wie bereits im Zusammenhang mit Fig. 1 erklärt) durch eine entsprechende Gestaltung der Einlauföffnung 20 mit Mundstück 29 sichergestellt.

**Patentansprüche**

1. Autotransfusionsgerät für Blut oder der gleichen Körperflüssigkeit, bestehend aus
a) einem evakuierbaren, unterdruckstabilen, ein Bodenteil (4) und ein Deckelteil (3) aufweisenden Behälter (5) mit
a1) einer im Deckelteil angeordneten Einlauföffnung (20) für die Körperflüssigkeit,
a2) einer Auslauföffnung (24) für die Körperflüssigkeit mit einem die Auslauföffnung abdeckenden Sieb (25) sowie
a3) einer Öffnung (9) zum Herstellen einer Gasströmungsverbindung zu einem Raum mit einem vom Behälterinneren verschiedenen Gasdruck,
dadurch gekennzeichnet, daß
b) auch die Auslauföffnung (24) im Deckelteil (3) angeordnet ist,
c) die Öffnung (9) im Bodenteil (4) angeordnet ist und
d) eine durch Druck verformbare, gegebenenfalls beutel- oder ballonförmige für die Körperflüssigkeit undurchlässige Membran (16)
d1) zwischen dem Boden- und dem Deckelteil randseitig gasdicht festgelegt ist,
d2) den Behälter (5) in einen gasgefüllten Bodenraum (13) und einen demgegenüber abgedichteten Deckelraum (19) zum Aufnehmen der Körperflüssigkeit unterteilt,
d3) unter dem Einfluß eines Fluiddruckes sowohl an die Innenkontur (1) des Deckelteils (3), als auch an die Innenkontur (2) des Bodenteils (4) im wesentlichen anlegbar ist und
e) das Sieb (25) mit Abstand vor der Auslauföffnung (24), insbesondere lösbar, gehalten ist und einen Raum (26) zum Zurückhalten auf der Körperflüssigkeit aufschwimmender, durch das Sieb mitgeführter Stoffe definiert.

2. Gerät nach Anspruch 1, dadurch gekennzeichnet, daß der Behälter (5) mit der Membran (16) als einteiliger Einmal-Behälter, insbesondere aus Kunststoff, ausgebildet ist.

3. Gerät nach Anspruch 1 oder 2, dadurch gekennzeichnet, daß das Deckelteil (3) lösbar an dem Bodenteil (4) befestigt, insbesondere verschraubt, ist.

4. Gerät nach einander Ansprüche 1 bis 3, dadurch gekennzeichnet, daß ein Randabschnitt (15) der Membran (16) zwischen Deckel- und Bodenteil eingeklemmt ist.

5. Gerät nach einander Ansprüche 1 bis 4, dadurch gekennzeichnet, daß ein verstärkter Rand (14) der Membran (16) in einer Ringnut (17) des Deckelrandes gasdicht gehalten ist.

6. Gerät nach Anspruch 1, dadurch gekennzeichnet, daß die Membran (16) ein aufblasbarer, mit seinem Mündungsrand (12) gegenüber dem Rand der Öffnung (9) gasdicht festgelegter Ballon ist.

7. Gerät nach einander Ansprüche 1 bis 6, dadurch gekennzeichnet, daß die Einlauföffnung (20) tangential zu der Innenwand (21) des Deckelteils (3) mündet.

8. Gerät nach einander Ansprüche 1 bis 7, dadurch gekennzeichnet, daß die Einlauföffnung (20) aus einem T-förmigen Mundstück besteht.

9. Gerät nach einander Ansprüche 1 bis 7, dadurch gekennzeichnet, daß die Einlauföffnung (20) aus einem trompetenartig erweiterten Mundstück (29) besteht.

10. Gerät nach einem der Ansprüche 1 bis 9, dadurch gekennzeichnet, daß das Bodenteil (4) einen Fuß (6) aufweist.

11. Vefahren zum betreiben eines Autotransfusionsgerätes für Blut oder der gleichen Körperflüssigkeit, bestehend aus
a) einem evakuierbaren, unterdruckstabilen, ein Bodenteil (4) und ein Deckelteil (3) aufweisenden Behälter (5) mit
a1) einer im Deckelteil angeordneten Einlauföffnung (20) für die Körperflüssigkeit,
a2) einer Auslauföffnung (24) für die Körperflüssigkeit mit einem die Auslauföffnung abdeckenden Sieb (25) sowie
a3) einer Öffnung (9) zum Herstellen einer Gasströmungsverbindung zu einem Raum mit einem vom Behälterinneren verschiedenen Gasdruck, bei dem
b) auch die Auslauföffnung (24) im Deckelteil (3) angeordnet ist,
c) die Öffnung (9) im Bodenteil (4) angeordnet ist und
d) eine durch Druck verformbare, gegebenenfalls beutel- oder ballonförmige für die Körperflüssigkeit undurchlässige Membran (16)
d1) zwischen dem Boden- und dem Deckelteil randseitig gasdicht festgelegt ist,

d2) den Behälter (5) in einen gasgefüllten Bodenraum (13) und einen demgegenüber abgedichteten Deckelraum (19) zum Aufnehmen der Körperflüssigkeit unterteilt,

d3) unter dem Einfluß eines Fluiddruckes sowohl an die Innenkontur (1) des Deckelteils (3), als auch an die Innenkontur (2) des Bodenteils (4) im wesentlichen anlegbar ist und

e) das Sieb (25) mit Abstand vor der Auslauföffnung (24), insbesondere lösbar, gehalten ist und einen Raum (26) zum Zurückhalten auf der Körperflüssigkeit aufschwimmender, durch das Sieb mitgeführter Stoffe definiert, wobei bei nach oben gerichtetem Deckelteil während einer ersten Arbeitsphase im Deckelraum zunächst ein Unterdruck angelegt wird, daß nach Beendigung des Unterdrucklegens der unterdruckstabile Behälters auf den Kopf gedreht wird und daß anschließend die Membran während einer zweiten Arbeitsphase im Richtung auf das Sieb hin verformt wird.

**Revendications**

1. Dispositif d'autotransfusion pour du sang ou un liquide corporel analogue, comprenant

a) un récipient (5) pouvant être mis sous un vide stable, comportant un fond (4) et un couvercle (3) ayant

a1) une ouverture d'arrivée (20) prévue dans le couvercle pour le liquide corporel,

a2) une ouverture de sortie (24) pour le liquide corporel munie d'un tamis (25) couvrant ladite ouverture ainsi que

a3) un orifice (9) pour établir une liaison de flux de gaz vers un espace dans lequel la pression du gaz est différente de celle régnant à l'intérieur du récipient, caractérisé en ce que

b) l'ouverture de sortie (24) est également prévue dans le couvercle (3),

c) l'orifice (9) est prévu dans le fond (4) et

d) qu'une membrane (16) étanche au liquide corporel, le cas échéant ayant la forme d'un sac ou d'un ballon, déformable par pression

d1) est fixée sur le côté du bord de manière étanche au gaz entre le fond et le couvercle,

d2) ladite membrane divisant le récipient (5) en un espace de fond (13) rempli de gaz et un espace de couvercle (19) rendu étanche par rapport à ce dernier pour recevoir le liquide corporel,

d3) ladite membrane pouvant se mettre sensiblement contre le contour intérieur (1) du couvercle (3) ainsi que contre le contour intérieur (2) du fond (4) sous l'influence de la pression du fluide et

e) lorsque le couvercle est dirigé vers le haut, on applique d'abord un vide pendant une première phase de travail dans l'espace de couvercle, que après avoir terminé l'application du vide le récipient ayant un vide stable est retourné la tête en bas, et qu'ensuite, dans une seconde phase de travail, la membrane est déformée en direction du tamis.

2. Dispositif selon la revendication 1, caractérisé en ce que le récipient (5) comprenant la membrane (16) est réalisé en tant que récipient d'une seule pièce servant une seule fois, notamment en matière plastique.

3. Dispositif selon l'une des revendications 1 ou 2, caractérisé en ce que la couvercle (3) est fixé amovible sur le fond (4), notamment par vissage.

4. Dispositif selon l'une des revendications 1 à 3, caractérisé en ce qu'un segment du bord (15) de la membrane (16) est maintenu serré entre le couvercle et le fond.

5. Dispositif selon l'une des revendications 1 à 4, caractérisé en ce qu'un bord renforcé (14) de la membrane (16) est guidé de façon étanche au gaz dans une rainure annulaire (17) du bord du couvercle.

6. Dispositif selon la revendication 1, caractérisé en ce que la membrane (16) est constituée par un ballon gonflable fixé de façon étanche au gaz par son bord d'ouverture (12) en face du bord de l'orifice (9).

7. Dispositif selon l'une des revendications 1 à 6, caractérisé en ce que l'ouverture d'arrivée (20) débouche tangentiellement par rapport à la paroi intérieure (21) du couvercle (3).

8. Dispositif selon l'une des revendications 1 à 7, caractérisé en ce que l'ouverture d'arrivée (20) affecte la forme d'une embouchure en forme de T.

9. Dispositif selon l'une des revendications 1 à 7, caractérisé en ce que l'ouverture d'arrivée (20) affecte la forme d'une embouchure (29) élargie en forme de trompette.

10. Dispositif selon l'une des revendications 1 à 9, caractérisé en ce que le fond (4) est muni d'un socle (6).

11. Procédé pour la mise en oeuvre d'un dispositif d'autotransfusion pour le sang ou un liquide corporel analogue, comprenant

a) un récipient (5) pouvant être mis sous un vide stable, comportant un fond (4) et un couvercle (3) ayant

a1) une ouverture d'arrivée (20) prévue dans le couvercle pour le liquide corporel,

a2) une ouverture de sortie (24) pour le liquide corporel munie d'un tamis (25) couvrant ladite ouverture ainsi que

a3) un orifice (9) pour établir une liaison de flux de gaz vers un espace dans lequel la pression de gaz est différente de celle régnant à l'intérieur du récipient, dans lequel

b) l'ouverture de sortie (24) est également prévue dans le couvercle (3),

c) l'orifice (9) est prévu dans le fond (4) et

d) qu'une membrane (16) étanche au liquide corporel, le cas échéant, ayant la forme d'un sac ou d'un ballon, déformable par pression

d1) est fixée sur le côté du bord de manière étanche au gaz entre le fond et le couvercle,

d2) ladite membrane divisant le récipient (5) en un espace de fond (13) rempli de gaz et un espace de couvercle (19) rendu étanche par rapport à ce dernier pour recevoir le liquide corporel,

d3) ladite membrane pouvant se mettre sensiblement contre le contour intérieur (1) du couvercle (3) ainsi que contre le contour intérieur (2) du fond (4) sous l'influence de la pression du fluide et

e) lorsque le couvercle est dirigé vers le haut, on applique d'abord un vide dans l'espace du couvercle pendant une première phase de travail, que après avoir terminé l'application du vide le récipient mis sous un vide stable est retourné la tête en bas, et qu'ensuite, dans une seconde phase de travail, la membrane est déformée en direction du tamis.

**Claims**

1. Autotransfusion apparatus for blood or equivalent body fluid, consisting of
   a) an evacuable container (5) which is stable under low pressure and which has a base part (4) and a lid part (3), with
   a1) an inlet opening (20) for the body fluid, arranged in the lid part,
   a2) an outlet opening (24) for the body fluid, with a filter (25) covering the outlet opening, and
   a3) an opening (9) for producing a gas flow connection to a chamber which has a different gas pressure from that of the interior of the container,
   characterised in that
   b) the outlet opening (24) is also arranged in the lid part (3),
   c) the opening (9) is arranged in the base part (4), and
   d) a pressure deformable diaphragm (16), optionally in the form of a bag or balloon, and which is impermeable to the body fluid
   d1) is peripherally secured in a gastight manner, between the base and lid parts,
   d2) divides the container (5) into a gas-filled base chamber (13) and a lid chamber (19) sealed relative thereto, for receiving the body fluid,
   d3) under the influence of a fluid pressure, can be applied substantially not only against the inner contour (1) of the lid part (3), but also against the inner contour (2) of the base part (4), and
   e) the filter (25) is held spaced at a distance before the outlet opening (24), in particular in a detachable manner, and defines a chamber (26) for retaining substances suspended in the body fluid and which are carried along therewith through the filter.

2. Apparatus according to claim 1, characterised in that the container (5), with the diaphragm (16), is constructed as an integral, one-use container, especially of plastics material.

3. Apparatus according to claim 1 or 2, characterised in that the lid part (3) is detachably secured on the base part (4), especially screwed on.

4. Apparatus according to any one of the claims 1 to 3, characterised in that an edge section (15) of the diaphragm (16) is clamped between the lid and the base parts.

5. Apparatus according to any one of the claims 1 to 4, characterised in that a reinforced edge (14) of the diaphragm (16) is held in a gastight manner in an annular groove (17) of the edge of the lid.

6. Apparatus according to claim 1, characterised in that the diaphragm (16) is an inflatable balloon which is secured in a gastight manner with its orifice edge (12) opposite the edge of the opening (9).

7. Apparatus according to any one of the claims 1 to 6, characterised in that the inlet opening (20) opens tangentially to the inner wall (21) of the lid part (3).

8. Apparatus according to any one of the claims 1 to 7, characterised in that the inlet opening (20) consists of a T-shaped mouthpiece.

9. Apparatus according to any one of the claims 1 to 7, characterised in that the inlet opening (20) consists of a trumpet-shaped widened mouthpiece (29).

10. Apparatus according to any one of the claims 1 to 9, characterised in that the base part (4) has a foot (6).

11. Method for operating an autotransfusion apparatus for blood or equivalent body fluid, consisting of
   a) an evacuable container (5) which is stable under low pressure and which has a base part (4) and a lid part (3), with
   a1) an inlet opening (20) for the body fluid, arranged in the lid part,
   a2) an outlet opening (24) for the body fluid, with a filter (25) covering the outlet opening, and
   a3) an opening (9) for producing a gas flow connection to a chamber which has a different gas pressure from that of the interior of the container, in which
   b) the outlet opening (24) is also arranged in the lid part (3),
   c) the opening (9) is arranged in the base part (4), and
   d) a pressure deformable diaphragm (16), optionally in the form of a bag or balloon, and which is impermeable to the body fluid
   d1) is peripherally secured in a gastight manner, between the base and lid parts,
   d2) divides the container (5) into a gas-filled base chamber (13) and a lid chamber (19) sealed relative thereto, for receiving the body fluid,
   d3) under the influence of a fluid pressure, can be applied substantially not only against the inner contour (1) of the lid part (3), but also against the inner contour (2) of the base part (4), and
   e) the filter (25) is held spaced at a distance before the outlet opening (24), in particular in a detachable manner, and defines a chamber (26) for retaining substances suspended in the body fluid and which are carried along therewith through the filter, wherein during a first operating phase, the lid part is on top and a low pressure is first applied in the lid chamber; that after the termination of the application of low pressure, the container, stable under low pressure, is inverted; and that subsequently, during a second operating phase, the diaphragm is deformed towards the filter.

Fig.1

Fig.2

# Fig. 3

Fig.4

Fig.5

4